# EUROPÄISCHE PATENTSCHRIFT

(11) **EP 3 041 574 B1**
(45) Veröffentlichungstag und Bekanntmachung des Hinweises auf die Patenterteilung: **19.05.2021**
(21) Anmeldenummer: 14761350.9
(22) Anmeldetag: 05.09.2014
(51) Int. Cl.: A61N 2/02, A61N 1/04, A61N 1/05, A61N 1/36, A61N 1/32

(54) **SYSTEM ZUR NICHT-INVASIVEN NEURONALEN STIMULATION**
SYSTEM FOR NON-INVASIVE NEURONAL STIMULATION
SYSTÈME POUR UNE STIMULATION NEURONALE NON INVASIVE

(30) Priorität: 06.09.2013 DE 102013014875; 23.05.2014 DE 102014007645; 11.08.2014 DE 102014011867
(43) Veröffentlichungstag der Anmeldung: 13.07.2016
(73) Patentinhaber: Neuromodtronic GmbH, 14473 Potsdam (DE)
(72) Erfinder: WUNDRICH, Ingo, 14532 Kleinmachnow (DE); WARSCHEWSKE, Udo, 12249 Berlin (DE)
(74) Vertreter: Meissner Bolte Partnerschaft mbB
(86) Internationale Anmeldenummer: PCT/EP2014/068945
(87) Internationale Veröffentlichungsnummer: WO 2015/032898

(56) Entgegenhaltungen:
- WO-A1-2012/000546
- CN-A- 101 491 715
- DE-A1-102011 120 213
- US-A1- 2004 131 998
- Bernhard A Sabel ET AL: "Non-invasive alternating current stimulation improves vision in optic neuropathy", Restorative neurology and neuroscience, 1. Januar 2011 (2011-01-01), Seiten 493-505, XP055153090, Netherlands DOI: 10.3233/RNN-2011-0624 Gefunden im Internet: URL:http://www.ncbi.nlm.nih.gov/pubmed/221 24039

## Beschreibung

Die Erfindung betrifft ein System zur nicht-invasiven elektrischen und/oder magnetischen Stimulation, insbesondere zur neuronalen Stimulation eines Patienten.

Nach dem Stand der Technik sind derzeit im Wesentlichen zwei grundlegende Verfahren für die elektrische Hirnstimulation zu unterscheiden, nämlich die invasive und die nicht-invasive elektrische Hirnstimulation.

In der klinischen Neurologie gewinnen Verfahren zur Hirnstimulation als vielversprechende Ergänzung zu konventionellen chirurgischen oder pharmakologischen Interventionsmaßnahmen zunehmend an Bedeutung. Dies betrifft zunächst die gut etablierten invasiven Verfahren zur Tiefenhirnstimulation (Deep Brain Stimulation) über implantierte "Hirnschrittmacher", die derzeit vor allem zur effektiven Behandlung von Bewegungsstörungen (z. B. bei Parkinson, essentiellem Tremor und Dystonie) eingesetzt, aber auch zur Behandlung zahlreicher anderer neurologischer Störungen untersucht werden.

Darüber hinaus wurden in den letzten Jahren bemerkenswerte Behandlungserfolge bei der Behandlung neurologischer Schäden durch den gezielten Einsatz nicht-invasiver, magnetischer oder elektrischer, transkranialer Stimulationsverfahren erreicht, die offensichtlich in der Lage sind, die der Regeneration zugrundeliegende neuronale Plastizität wirksam zu unterstützen.

Eine der quantitativ wichtigsten Ursachen für Hirnschäden liegt in der Indikation Schlaganfall begründet. Alleine in Deutschland ereignen sich jedes Jahr ca. 200.000 Schlaganfälle. Damit sind ca. 65.000 Todesfälle assoziiert, was den Schlaganfall zur dritthäufigsten Todesursache in Deutschland macht. Der überwiegende Teil der Überlebenden ist von gravierenden neurologisch bedingten Beeinträchtigungen betroffen; dies betrifft vor allem die motorischen Funktionen (Lähmungserscheinungen), die Sprachfunktion und die Sehfunktion (z. B: Gesichtsfeldeinschränkungen).

Während der Schwerpunkt der heute gebräuchlichen Rehabilitierungsmaßnahmen auf der Wiederherstellung bzw. Verbesserung der motorischen und sprachlichen Fertigkeiten liegt, um den Patienten zumindest aus einer permanenten Pflegebedürftigkeit herauszuhalten, stehen rehabilitive Maßnahmen zur Verbesserung der häufig stark beeinträchtigten visuellen Fähigkeiten kaum zur Verfügung.

Die spontane Selbstheilung aufgrund neuroplastischer Vorgänge verläuft individuell sehr verschieden, gleichzeitig sind die beschriebenen sensorischen Einschränkungen mit einem hohen Leidensdruck für die betroffenen Patienten verbunden. Aufgrund dieser Tatsache und vor dem Hintergrund der hohen Patientenzahlen besteht ein außerordentlich großes Interesse an neuen und effektiven Behandlungskonzepten, vor allem wenn diese nichtinvasiv sind bzw. ein günstiges Nutzen-Risiko-Verhältnis aufweisen. In diesem Zusammenhang erscheinen elektrische oder magnetische, transkraniale Stimulationsverfahren als ein besonders interessanter Ansatz.

Die nicht-invasiven Verfahren auf dem Gebiet der elektrischen Neurostimulation von Hirnarealen sind vorrangig die sog. transkranialen Verfahren zur nicht-invasiven Therapie des Gehirns. Die therapeutische Stimulation erfolgt hierbei ohne einen interventionellen Eingriff in das Gehirn. Stattdessen wird die Behandlung "transkranial", d.h. von außerhalb des Schädels "durch den Schädel hindurch" durchgeführt. Hinsichtlich des applizierten Stroms wird innerhalb der elektrischen Stimulationsverfahren zwischen der transkranialen Gleichstromstimulation (tDCS), der transkranialen Stimulation mit randomisierten hochfrequenten Stromsignalen (tRNS - transcranial High-Frequency Random Noise Stimulation) und der transkranialen Wechselstromstimulation (tACS) unterschieden. Mit der transkranialen elektrischen Hirnstimulation können nachweislich anhaltende neuroplastische Veränderungen erreicht werden, die mit erhöhter elektrischer Aktivität in neuronalen Strukturen oder der Synchronisation der sogenannten CSTC-Regelkreise (cortico-striato-thalamo-cortikale loop model) einhergeht. Im Falle der transkranialen Gleichstromstimulation wird davon ausgegangen, dass durch die Stimulation das Membranpotenzial kortikaler Neuronen und die Aktivität ("firing rate") einzelner Neuronen moduliert werden kann. Resultiert eine erhöhte Aktivität, so entspricht dies einer Langzeit-Potenzierung, bei einer reduzierten Aktivität einer Langzeit-Depression der neuronalen Signalübertragung. Die tRNS stellt eine komplementäre und neuere Methode der nicht-invasiven Hirnstimulation dar. Im Gegensatz zur tDCS beinhaltet die tRNS keine Gleichstromkomponente und es gibt keinen Hinweis darauf, dass inhibitorische Nachwirkungen induziert werden können. Neben einer potentiell größeren Sicherheit wird ein weiterer Vorteil darin gesehen, dass eine Depolarisation von Zellen jeglicher Orientierung unabhängig von der Flussrichtung des Stroms erfolgt. Die Wirkung wird auf eine synaptische Signalverstärkung, verbesserte Signalweiterleitung und erhöhte neuronale Aktivität durch Rauscheintrag nach dem Prinzip der "stochastischen Resonanz" zurückgeführt.

Während mittels tDCS und tRNS nach aktuellem Kenntnisstand die Wahrscheinlichkeit der neuronalen Signalabgabe ("Firing rate probability") moduliert werden kann, handelt es sich bei der transkraniale Wechselstromstimulation (tACS) um ein Verfahren, das offensichtlich spezifische oszillatorische Aktivitäten innerhalb neuronaler Strukturen induzieren oder beeinflussen kann, die mit der Erregbarkeit von Pyramidenzellen und der Synchronisation von CSTC-Regelkreise assoziiert sind. Dabei wird davon ausgegangen, dass die Methode direkt mit Brain State-spezifischen Oszillationen wechselwirkt (Als Brain State wird in diesem Zusammenhang eine mentaler Zustand verstanden, der mit der Synchronisation bestimmter neuronaler Verbünde einhergeht).

Bei der transkranialen elektrischen Hirnstimulation werden zwei oder mehrere Elektroden auf bzw. unterhalb der Kopfhaut des Patienten platziert und für eine begrenzte Zeitdauer von bis zu 40 Minuten zur Stimulation der neuronalen Schaltkreise im Gehirn mit einem Gleich- bzw. Wechselstrom beaufschlagt.

Bei den Verfahren der Gehirnstimulation kann zusätzlich zwischen direkten und indirekten Verfahren unterschieden werden. Bei den direkten Verfahren werden kortikale bzw. subkortikale Strukturen des Gehirns unmittelbar durch den applizierten Stimulus beeinflusst während bei den indirekten Verfahren die Stimulation des Gehirns mittelbar über die Reizung der Hirnnerven (z.B. Sehnerv, Vagusnerv) oder die Reizung peripherer Nerven erfolgt.

DE 10 2011 12023 A1, beschreibt eine Vorrichtung zur transkraniellen Wechselstromsimulation zur Behandlung von Störungen der motorischen/kognitiven Funktionen im Gehirn.

Aus dem Stand der Technik ist weiterhin ein System zur nicht-invasiven Elektrostimulation bekannt, das auf dem Prinzip der rtACS) basiert. Dabei erfolgt eine elektrische Hirnstimulation mit Wechselstrom nicht transkranial sondern mittelbar über die Retina und den Sehnerv (retinofugale Stimulation). Die Elektroden werden hierfür unmittelbar um die Augen platziert. In den bisherigen Studien erweist sich das System bei einem Teil der Patienten (Responder) bei der Behandlung neurologischer Sehstörungen als hochgradig effektiv. Die Behandlung eignet sich nicht nur für Schlaganfallpatienten, sondern auch für andere Formen von Sehstörungen, die auf neurologische Schädigungen des Gehirns und der Sehbahn zurückzuführen sind (z. B. bei degenerativen Erkrankungen oder traumatischen Schädigungen des Gehirns). Das Potenzial dieses Behandlungskonzepts erscheint immens, allerdings ist die bislang beobachtete Wirksamkeit individuell sehr verschieden, es wird dementsprechend zwischen "Respondern" und "Non-Respondern" unterschieden. Nach dem derzeitigen Stand der Neurowissenschaft ist davon auszugehen, dass eine individuell auf den jeweiligen Patienten optimierte Stimulationsbehandlung zu einer sehr viel besseren Wirksamkeit führt, womit die Voraussetzung für einen flächendeckenden Einsatz in der Neurorehabilitation geschaffen würde. Da die zugrundeliegenden Mechanismen (ähnlich wie bei den anderen Verfahren der elektrischen Hirnstimulation) nur unzureichend verstanden sind, ist die Optimierung der Behandlungsverfahren nur langsam fortgeschritten.

Es ist daher eine Aufgabe der vorliegenden Erfindung, ein System und ein Verfahren zur elektrischen und/oder magnetischen Stimulation bereitzustellen, bei dem die Stimulation dynamisch an den Patienten angepasst und daher optimiert werden kann.

Diese Aufgabe wird gelöst durch ein System nach Anspruch 1. Vorteilhafte Ausgestaltungen sind in den Unteransprüchen beschrieben.

Insbesondere wird ein System bereitgestellt, das umfasst:
- einen Signalgenerator zur Erzeugung eines Stimulationssignals, insbesondere eines Wechselstrom-Stimulationssignals,
- eine Applikationsvorrichtung zum Applizieren des Stimulationssignals, insbesondere in einem Bereich am oder unmittelbar um den Sehnerv,
- eine Ableitung zum Ableiten eines Messsignals, insbesondere eines EEG-Signals,
- eine Biomarker-Berechnungseinheit zum Berechnen eines Biomarkers basierend auf dem Messsignal,
- eine Optimierungseinheit zur Aktualisierung des Optimierungskriteriums, d. h. zum Optimieren des Werts des Biomarkers durch Variation des Stimulationssignals.

Es versteht sich, dass das Messsignal eine Vielzahl von Einzelsignalen umfassen kann, z.B. ausgehend von einer Vielzahl von EEG-Elektroden. Ebenso kann das Stimulationssignal eine Vielzahl von Einzelsignalen umfassen.

Die magnetische Stimulation kann auf Basis der bekannten transkranialen magnetischen Stimulation (TMS) erfolgen.

Der Biomarker ist in diesem Zusammenhang ein Element aus einem Merkmalsraum, der den Zustand eines Patienten oder speziell dessen Gehirns hinsichtlich einer körperlichen Funktionalität bzw. eines Krankheitsbildes hinreichend beschreibt. Biomarker können entweder krankheits- oder therapiebezogen sein. Ein krankheitsbezogener Biomarker gibt Auskunft über eine drohende oder eine in einem (sehr) frühen Stadium bereits bestehende Erkrankung, wohingegen ein therapiebezogener Biomarker angibt, ob und wie die Therapie auf einen bestimmten Patienten wirkt, und wie dessen Organismus diese umsetzen wird. Diese Unterscheidung ist für die Zielsetzung bei der Optimierung von Bedeutung.

Der Biomarker kann ein- oder mehrdimensional sein. Die Merkmalsgrößen können zusätzlich gewichtet sein. Bei der Wichtung kann es sich um eine Menge von statistischen Kenngrößen handeln. Die erfindungsgemäße Optimierung kann dabei darin bestehen, bestimmten Werten, respektive einem bestimmten Zustand möglichst nahe zu kommen. In Ausgestaltungen der Erfindung handelt es sich dabei nicht notwendigerweise um das Erreichen lokaler oder globaler Extrema.

In einer weiteren Ausgestaltung kommt es darauf an, die Annäherung an Risikozustände im Raum des Biomarkers zu vermeiden, sei es durch Umgehen dieser Bereiche oder Beenden bzw. Neustart der Therapie bei auswegloser Annäherung.

In einer weiteren Ausgestaltung erhält der Patient während der Therapie, also der Optimierung des Biomarkers, nach Maßgabe des aktuellen Biomarkers vom System Instruktionen, z.B. schriftlich oder akustisch. Ein Ziel derartiger Instruktionen ist z.B. eine Änderung des Aufmerksamkeitszustands des Patienten. Dadurch werden z.B. aufmerksamkeitszustandspezifische Messdaten erzeugt, die für den Biomarker relevant sein können. Hierbei ergibt sich für jeden Aufmerksamkeitszustand ein eigener Biomarkerraum, die durch gelernte Abbildungen in einen anderen abgebildet werden kann.

Die Aufmerksamkeitszustände des Gehirns können von Schlaf oder Entspannung bis zu hoher Konzentration bei der Lösung schwieriger Aufgaben reichen. Dies schließt auch vorgegebene Bewegungsabläufe mit ein, um beispielsweise die transkraniale Stimulation des Motorcortexes zu unterstützen.

Jegliche Approximation an einen Ziel- oder Idealzustand wird im Folgenden als Optimierung bezeichnet. Die praktische Umsetzung kann z.B. durch Optimierung im Sinne einer Extremwert-Bestimmung einer Abstands-Funktion zwischen Ziel-Werten und gemessenen Biomarker-Werten erfolgen.

Maßgeblich für die Struktur des Raums in dem die Optimierung erfolgen soll ist das Vorhandensein einer bedeutsamen Metrik, also dem auf den

Zustand des Patienten bzw. Verlauf der Therapie bezogene Topologie von Biomarken.

Bei den Applikationsvorrichtungen kann es sich z.B. um Elektroden zur elektrischen Stimulation und/oder um Spulen zur magnetischen Stimulation handeln.

Erfindungsgemäß kann eine Überlagerung von Gleich- und Wechselstrom-Signalen mit einem künstlichen Rauschen, insbesondere mit weißem und gefiltertem Rauschen, erfolgen.

In einer weiteren Ausgestaltung der Erfindung werden Wechselstromsignalformen durch Rauschen überlagert, das über die gesamte gültige Zeitachse oder gefenstert als Bursts vorliegen kann.

In einer weiteren Ausgestaltung können frei vom Klassifikationsschema "Gleichstrom/ Wechselstrom/ Rauschen" allgemeine amplitudenbeschränkte Signale mit Abtastraten bis zu beispielsweise 10MHz während einer Therapie appliziert werden.

Das erfindungsgemäße System ermöglicht es, dass während des Stimulations-Vorgangs die Stimulation angepasst wird, um ein maximales Ansprechen zu erzielen. Durch die automatische Erfassung des Messsignals und die Bestimmung des Biomarkers zeitgleich oder minimal zeitversetzt zur Stimulation kann das Stimulationssignal innerhalb kurzer Zeit so variiert werden, dass aus einem Non-Responder (bezogen auf ein erstes Stimulationssignal) ein Responder wird (bezogen auf das variierte Stimulationssignal).

Es wird somit eine patientenindividuelle automatische Optimierung der Elektrostimulation während der Behandlung ermöglicht, indem ein zuvor entwickelter, für die Plastizitätsunterstützung relevanter Biomarker beobachtet und über eine Adaptation der Stimulation, z.B. über eine Variation der Stimulationsparameter, maximiert wird. Damit kann eine starke Erhöhung des Anteils der Responder bzw. Super-Responder erreicht und letztlich ein stark verbessertes nicht-invasives Elektrostimulationsverfahren zur wirksamen Behandlung neurologischer Störungen, insbesondere Sehstörungen, geschaffen werden, das unter anderem in der Rehabilitierung von Schlaganfallpatienten völlig neue Maßstäbe setzt.

Das Optimierungskriterium basiert auf der Unterscheidung zwischen krankheits- und therapiebezogenen Biomarkern. Im ersten Fall gibt es für den gesunden Menschen eine Normregion im Biomarkerraum, deren Erreichen Heilung verspricht. Ziel ist es, diese Normregion zu erreichen bzw. bezüglich einer bedeutsamen Metrik ihr möglichst nahe zu kommen. Bei therapiebezogenen Biomarkern sind ebenfalls Zielregionen im Biomarkerraum denkbar, die mit einem besonders guten Ansprechen der Patienten auf die Therapie einhergehen. Dabei kann der therapiebezogene Biomarker prinzipiell als Selektionskriterium für die Anwendbarkeit der Therapie verwendet werden und somit Hinweise auf einen möglichen Erfolg geben. Andererseits ist es in Vorbereitung mit zusätzlichen therapeutischen (z.B. pharmakologischer) Maßnahmen möglich, den Patienten in einen für die Therapie günstigeren Allgemeinzustand zu bringen, d.h. den Biomarker näher an eine Zielregion zu bringen.

In einer entsprechenden Ausgestaltung werden Zielregionen bewertbar gemacht durch einen Skalar, der Kriterien wie z.B. den therapeutischen Erfolg, das Ansprechen des Patienten auf die Therapie oder die Wahrscheinlichkeit des Erreichens berücksichtigt.

Der Lösungsansatz trägt der Tatsache Rechnung, dass mechanistische Grundlagen der Wiederherstellung der neurologischen Ausfälle und ihrer Unterstützung mittels transkranialer Stimulationsverfahren nur unzureichend verstanden sind und basiert daher auf einem weitestgehend datengetriebenen Vorgehen.

Für eine simultane, orts-, frequenz- und phasenspezifische Ableitung und Stimulation kann eine Elektrodenkappe mit frei programmierbaren Ableit- und Stimulationselektroden verwendet werden. Durch die Verwendung leitfähiger Textilien kann die hierfür erforderliche Formfreiheit zusätzlich verbessert werden.

Erfindungsgemäß wird somit ein geregeltes Stimulationssystem geschaffen, dass das Stimulationsparadigma anhand der aktuellen Zustandsparameter des Patienten selbständig durch Änderungen von Stimulationskenngrößen gezielt in Richtung eines optimalen Brain States treibt und somit die Therapie individuell an den Patienten anpasst, deren Wirksamkeit verbessert bzw. den relativen Anteil der Responder bzw. Super-Responder stark erhöht.

Die Stimulation kann dabei je nach Ausgestaltung nicht nur als retinofugale Stimulation, sondern, mittels einer flexiblen Konfiguration der Elektroden am Kopf, auch als transkraniale Elektrostimulation, z.B. im Bereich des visuellen Cortex erfolgen.

In einer Ausgestaltung der Erfindung ist vorgesehen, dass das System einen Rauschgenerator zur Erzeugung eines künstlichen Rauschens aufweist.

Somit kann die Möglichkeit geschaffen werden, neben der Nutzung diskreter Frequenzen innerhalb bestimmter Frequenzintervalle auch Rauschanteile in die Stimulation einzukoppeln, um zusätzlich den in der aktuellen Wissenschaft gut etablierten Einfluss des Rauschens in der neuronalen Plastizität zu nutzen.

Im Allgemeinen ist der Biomarker eine Menge von gewichteten Parametern, die für alle Datensätze in Bezug auf die Diagnose und/ oder das Therapieziel charakteristische Eigenschaften bzw. Merkmale aufweisen, die durch die Analyse des Messsignals unter Berücksichtigung verschiedener Signaleigenschaften (wie zum Beispiel Amplituden-, Frequenz-, Phasen- und/oder Ortsinformationen des Messsignals und oder komplexerer Abbildung dieser Signale, wie z.B. Kreuzkorrelationen, Wavelet-Koeffizienten oder klinisch bewertbare Teststatistiken) gewonnen werden.

In einer weiteren Ausgestaltung der Erfindung ist vorgesehen, dass die funktionale Abhängigkeit des Biomarkers durch maschinelles Lernen aus Trainingsdaten bestimmt wurden, insbesondere der Biomarker eine Funktion von Amplituden-, Frequenz-, Phasen- und/oder Ortsinformationen des Messsignals oder deren funktionalen Abbildungen ist. Die Gewichtungsparameter können ebenfalls durch maschinelles Lernen aus Trainingsdaten bestimmt werden. Eine Ausführungsform eines solchen Lernverfahrens besteht darin, dass die Messdaten für die Bestimmung des Biomarkers anhand bestimmter Kriterien segmentiert werden. Dies kann sowohl anhand zeitlicher Kriterien (z.B. Zeitpunkt vor und nach einem Therapieversuch) als auch anhand qualitativer Kriterien zur Bewertung des Therapieversuches (so z.B. anhand quantifizierbaren Merkmale wie z.B. Größe des Gesichtsfeldes, Sehschärfe, Farb- und Konturensehen) erfolgen. Innerhalb dieser Segmente lassen sich Trends und Korrelationen charakteristischer Merkmale der Messsignale ermitteln. Signifikanz und Streuung der gefundenen Merkmale können z.B. für die Wichtung der Merkmalsparameter herangezogen werden.

Da es sich bei den funktionalen Zusammenhängen zwischen dem Messsignal und einem aussagekräftigen Biomarker um komplizierte, nichtlinearen Zusammenhänge handeln kann und es sich z.B. bei EEG-Messsignalen um insgesamt sehr hochdimensionalen Daten handelt, können hierbei insbesondere fortgeschrittene Lernmethoden wie z.B. Support-Vektor-Maschinen (SVMs) zum Einsatz kommen.

Dabei kann ein Biomarkerraum von seinen topologischen Bedingungen beispielsweise als linearer Skalarproduktraum unter Berücksichtigung der Signifikanz seiner Dimensionen (darüber die Definition der Metrik) oder als nichtlineare Mannigfaltigkeit angesehen werden. Letztere können mit Methoden der nichtlinearen Dimensionalitätsreduktion wie z.B. Isomap oder Local Linear Embedding kartographiert werden.

In einer weiteren Ausgestaltung der Erfindung sind die Biomarkerräume für eine bestimmte Patientenpopulation nicht fest in das Gerät eingebaut, sondern sie werden aus den Therapieveriäufen in der Menge aller Patienten, die mit dem Gerät therapiert werden, gelernt. Dies wäre z.B. durch Kommunikation über ein Netzwerk von Computern in verbundenen Therapiezentren möglich.

In einer weiteren Ausgestaltung der Erfindung wird die Stimulationstherapie pharmakologisch unterstützt. Die Medikation kann vordefiniert sein oder sich aus dem Verlauf der Therapie bzw. der Optimierung des Biomarkers ergeben. Ein Realisierungspfad kann z.B. über eine Schnittstelle zu Infusionsvorrichtungen erfolgen.

Der Biomarker lässt sich in Echtzeit bzw. mit minimaler Zeitverzögerung aus dem Messsignal bestimmen. Somit kann in dieser Ausgestaltung der Erfindung ein Ansprechen des Biomarkers praktisch ohne Verzögerung festgestellt werden und somit auch ggf. sofort eine Anpassung des Stimulationssignals erfolgen.

In einer weiteren Ausgestaltung der Erfindung ist vorgesehen, dass das Stimulationssignal erzeugt wird durch einen programmierbaren Funktionsgenerator, der vorzugsweise in einem Field Programmable Gate Array (FPGA) implementiert ist.

In dieser Ausgestaltung ist Kern des Stimulations-Systems ein FPGA, über den pro Kanal eine Stromquelle separat gesteuert wird. Dabei kann vorgesehen sein, dass der Output über eine oder mehrere miteinander kombinierte digitale Tabellen definiert wird (z.B. 8000 Einträge a 16 bit pro Tabelle), die mit einer Abtastrate zwischen 100 Hz und 1 MHz ausgelesen werden.

Für die Kodierung der Amplitude reichen dabei 12 bzw. 14 bit, so dass die verbleibenden 4 bzw. 2 bit als Kommandos innerhalb der Stimulationstabelle ("wave table") interpretiert werden können. Derartige Kommandos können sein: End of Waveform, Switch Table (liest die Werte aus einer weiteren Tabelle); Ground (schaltet den Kanal auf Masse - somit können bei mehreren Kanälen nicht nur die Stromstärke sondern auch die Stromflussrichtung beeinflusst werden "stearing"); Tag (Senden eines TTL Signals/Zeitstempels, das vom EEG Verstärker aufgezeichnet wird), Repeat Until (Wiederholtes auslesen der Tabelle bis ein bestimmter Zustand eingetreten ist). Auf diese Art und Weise kann das funktionale Verhalten des Stimulators mit Stimulationsparadigma synchronisiert werden.

Eine Optimierung der Tabellenladezyklen kann durch zusätzliche Modulation/ Skalierung bestehender Tabellen hinsichtlich ihrer Amplitude bzw. Abtastrate oder durch Überlagerung mehrerer Tabellen erfolgen. In einer weiteren Ausführungsform der Erfindung können derartige Änderungen der Kurvenformen während der Ausführung einer aktuellen Tabelle durchgeführt werden. So kann z.B. mit einem Befehl: Scale Amplitude/Scale Sampling Rate (Amplitude bzw. Abtastrate wird neu skaliert) die Amplitude bzw. das Frequenzverhalten des Stimulationsparadigmas an einer vorab festgelegten Stelle der Stimulationssequenz (z.B. bei einem Nulldurchgang des Signals) mit einem Wert moduliert werden, der vorab in einem Register des Stimulators abgelegt wurde (d.h. asynchrone Steuerung des Stimulators). Ähnliches ist in einer weiteren Ausführung der Erfindung mit Pausen bzw. Unterbrechungen z.B. durch einen Befehl wie Scale Delay (Anzahl von Leerlaufsamples einer Tabelle) möglich, wodurch eine Phasenmodulation des Stimulationssignals z.B. in Bezug auf ein neuronales Messsignal möglich ist.

In dem programmierbaren Funktionsgenerator können verschiedene Kurvenformen als mögliche Stimulationssignale gespeichert sein. Somit kann die Optimierungseinheit zwischen verschiedenartigen Stimulationssignalen wechseln und in einem großen Parameterraum die optimalen Einstellungen für die Stimulation wählen oder die Stimulationssignale werden nichtparametrisch erzeugt und appliziert

In einer weiteren Ausgestaltung der Erfindung ist eine Erweiterung mit einer Vorrichtung zum Erzeugen physiologischer Reize (z.B. das Erzeugen diskreter Lichtreize in unterschiedlichen Sektoren des Gesichtsfeldes) und/oder einer Feedback-Eingabeeinrichtung (z.B. ein Handschalter, Touch Screen oder Drehregler) vorgesehen. Wobei die Einrichtung zum Erzeugen physiologischer Reize vorzugsweise mit der Messvorrichtung synchronisiert ist und die Feedback-Daten der Feedback-Eingabeeinrichtung vorzugsweise bei der Berechnung des Biomarkers berücksichtigt werden.

Dabei kann vorgesehen sein, dass der Biomarker, und somit in Abhängigkeit vom Biomarker auch das Stimulationssignal, in Abhängigkeit von Feedback-Daten, die der Patient über eine Feedback-Eingabeeinrichtung eingibt, festgelegt wird.

In einer weiteren Ausgestaltung der Erfindung werden akustische Stimuli z.B. über einen Kopfhörer zur Stimulation von auditorisch-relevanten und höherliegenden Gehirnarealen appliziert.

In einer weiteren Ausgestaltung der Erfindung werden komplexere optische/visuelle Reize (z.B. Gitter- oder Schachbrettmuster) mit einer o.g. Feedback-Eingabeeinrichtung kombiniert, um die Funktionalität höherer visueller Verarbeitung bei dem gegebenen Patienten bewerten und in die Optimierung des Biomarkers einfließen zu lassen. Diese Funktionalität umfasst z.B. räumliche Auflösung (Frequenz- und Modulationstiefe), Orientierung von Mustern, Bewegung, räumliches Sehen.

In einer weiteren Ausgestaltung der Erfindung dienen Stimuli der erwähnten Arten sowohl therapeutischen Zwecken als auch der Aktualisierung des Biomarkers durch die Messung entsprechend evozierter Potentiale.

In Ausführungsformen weist das Stimulationssignal einer einzelnen oder mehreren Elektroden nur Gleichstrom, Wechselstrom oder hochfrequentes Rauschen auf. In anderen Ausführungsformen werden an einer oder mehreren Elektroden Überlagerungen aus allen drei der möglichen Stromparadigmen appliziert.

In einer weiteren Ausgestaltung der Erfindung ist vorgesehen, dass die funktionelle Abhängigkeit des Biomarkers vom Messsignal durch maschinelles Lernen mit Trainingsdaten von Probanden bestimmt wird, insbesondere durch supervised clustering mit Support Vektor Maschinen, zusätzlich unterstützt von Datenbanken. Das Verfahren kann somit aus dem Datenpool aller Patienten und jedes neu hinzugekommenen Patienten "lernen" .

In dieser Ausgestaltung der Erfindung kann aus vorhandenen Probanden-daten (Responder und Non-Responder) mit Hilfe von Mustererkennungsverfahren (Supervised Clustering mit Support Vector Machines) ein relevanter EEG-Biomarkersatz - sozusagen ein "EEG-Fingerabdruck der Recovery des visuellen Systems" - abgeleitet werden, der die verschiedenen Merkmale des Messsignals (z.B. Frequenz-, Phasen und Ortsinformationen) berücksichtigt. Die Zuordnung der Probanden-Daten zu Respondern und Non-Respondern kann manuell, z.B. durch einen erfahrenen Arzt, erfolgen.

Basierend auf diesem beobachtbaren "EEG-Fingerabdruck" können dann besonders wirksame Algorithmen zur schnellen stochastischen Optimierung der Stimulationsparameter zum Einsatz kommen.

In einer weiteren Ausgestaltung der Erfindung werden nicht nur EEG-Merkmale in den Biomarkerraum abgebildet sondern auch weitere Messungen, wie z.B. EKG oder funktionelle Magnetresonanztomographie, oder bewusste Patientenrückkopplung (s.u.). Dies schließt diagnostische Tests wie Perimetrie oder Audiometrie ein.

In einer weiteren Ausgestaltung der Erfindung ist vorgesehen, dass während des Stimulationsverfahrens ein Optimierungsverfahren, insbesondere ein stochastisches Optimierungsverfahren, durchgeführt wird, um durch eine Variation von Parametern des Stimulationssignals den Biomarker zu maximieren.

Stochastische Optimierungsverfahren haben sich in Experimenten, bei der je nach Ausgestaltung eine sehr hohe Anzahl von zu variierenden Parametern des Stimulationssignals gegeben ist, besonders robust gegenüber lokalen Minima herausgestellt.

Erfindungsgemäß können verschiedene stochastische Optimierungsverfahren unterschiedlicher Klassen (z.B. Evolutionsstrategien, genetische Algorithmen, stochastic gradient descent) verwendet werden, deren Gemeinsamkeit darin besteht, dass Änderungen an den Stellgrößen vorgenommen und post-hoc bewertet wird, ob diese zu einer Verbesserung geführt haben. Ebenfalls anwendbar sind dabei Verfahren des active learning, die auf eine maximale Partitionierung des verbleibenden Suchraums abzielen, sowie Verfahren des reinforcement learning ("Belohnungsprinzip").

Dabei wird der Tatsache Rechnung getragen, dass die zur Optimierung nötigen Operationen nicht exakt vorausgeplant werden können, sondern die Planung sich auf Trajektorien im Biomarkerraum bzw. auf miteinander durch Abbildungen verbundenen Biomarkerräumen bezieht. Diese Trajektorien werden anhand von Profilen geplant, die z.B. dem Patienten möglichst wenig Interaktion abverlangen, mit möglichst wenig Energie auskommen (Schmerzreduktion) oder bestimmte Risiken besonders minimieren (in Abhängigkeit von zusätzlichen Erkrankungen, die nicht Gegenstand der Therapie sind wie z.B. Epilepsie).

Die Optimierung ist beendet, wenn z.B. der Rand einer Zielregion im Biomarkerraum erreicht ist, der Zentroid einer Zielregion im Biomarkerraum erreicht ist, eine weitere Optimierung in der geplanten Richtung unmöglich erscheint oder der Biomarker den Einzugsbereich einer Risikoregion nicht verlassen kann. Die Menge der Risikoregionen ist eine Menge zusammenhängender Mengen, in dem der Biomarker mit nachteiligen Effekten auf den Patienten zusammenhängt. Dies ist z.B. bei Beeinflussung von Funktionen wie Herzschlag oder dem Hervorrufen von Angstzuständen der Fall.

In einer weiteren Ausgestaltung der Erfindung ist vorgesehen, dass die Merkmale des Messsignals (z.B. die Amplituden-, Frequenz-, und/oder Phaseninformation) bestimmt werden in einem bestimmten Zeitbereich nach der Anbringung eines Pulses des Stimulationssignals, insbesondere in einem Zeitbereich von 10 ms bis 100 ms, vorzugsweise 30 ms bis 300 ms, nach der Anbringung eines Pulses des Stimulationssignals.

Während der Applikation eines Pulses des Stimulationssignals kann es bei den Messelektroden zu starken Artefakten kommen. Diese Artefakte können im Bereich von einigen Volt liegen, während das abzuleitende Messsignal u.U. nur in der Größenordnung von µV liegt. In diesen Fällen ist eine Artefakt-Unterdrückung bzw. -Filterung u.U. wenig erfolgversprechend und die während bzw. unmittelbar nach dem Stimulations-Puls gemessenen Signale werden bei der Berechnung des Biomarkers nicht berücksichtigt.

In anderen Ausgestaltungen der Erfindung werden alle oder zumindest einige der Merkmale des Messsignals kontinuierlich bestimmt, d.h. ohne zeitliche Beschränkung auf ein bestimmtes Zeitfenster.

In einer weiteren Ausgestaltung der Erfindung ist vorgesehen, dass eine Vielzahl von vordefinierten Stimulations-Sequenzen, insbesondere gekennzeichnet durch verschiedene Amplituden, Frequenzen, Kurvenformen und örtliche Verteilung, nacheinander appliziert werden und jeweils im Anschluss der Biomarker bestimmt wird, und anschließend mit der Stimulations-Sequenz weiter stimuliert wird, mit der der dem Therapieziel entsprechende optimale Wert des Biomarkers erzielt wird.

Hierbei handelt es sich um ein besonders einfaches Verfahren, um für einen Patienten aus einer Reihe von bekannten Stimulations-Sequenzen die auszuwählen, auf die dieser Patient am besten anspricht.

In einer weiteren Ausgestaltung der Erfindung ist vorgesehen, dass das künstliche Rauschen weißes Rauschen, und gefiltertes Rauschen (z.B. f-Rauschen, 1/f-Rauschen und/oder 1/f²-Rauschen) umfasst, wobei in Abhängigkeit von dem Biomarker zwischen verschiedenen Arten des künstlichen Rauschens gewechselt wird.

In einer weiteren Ausgestaltung der Erfindung ist vorgesehen, dass erste Signale mit hohem Rauschanteil, insbesondere einem Rauschanteil von über 10%, vorzugsweise über 50% verglichen zum Trägersignal, und zweite Signale mit niedrigem Rauschanteil, insbesondere einem Rauschanteil von unter 10%, vorzugsweise unter 2%, erzeugt werden und die ersten und zweiten Signale in unterschiedlichen Regionen des Patienten und/oder mit unterschiedlichen Elektroden und/oder Spulen appliziert werden.

## Patentansprüche

1. System zur nicht-invasiven elektrischen und/oder magnetischen neuronalen Stimulation, umfassend:
- einen Signalgenerator zur Erzeugung eines Wechselstrom-Stimulationssignals,
- eine Applikationsvorrichtung konfiguriert zum Applizieren des Stimulationssignals in einem Bereich am oder unmittelbar um den Sehnerv,
- eine Ableitung zum Ableiten eines Messsignals,
- eine Biomarker-Berechnungseinheit konfiguriert zum Berechnen eines Biomarkers basierend auf dem Messsignal, wobei der Biomarker eine Menge von gewichteten Parametern ist, für die alle Datensätze in Bezug auf eine Diagnose und/oder ein Therapieziel charakteristische Eigenschaften bzw. Merkmale aufweisen,
- eine Einheit konfiguriert zur Umsetzung eines stochastischen Optimierungsverfahrens, zum Optimieren des Werts des Biomarkers durch Variation des Stimulationssignals.

2. System nach Anspruch 1,
**gekennzeichnet durch**
einen Rauschgenerator zur Erzeugung eines künstlichen Rauschens als Stimulationssignal.

3. System nach einem der vorherigen Ansprüche,
**dadurch gekennzeichnet, dass**
das Stimulationssignal erzeugt wird durch einen programmierbaren Funktionsgenerator.

4. System nach einem der vorherigen Ansprüche,
**dadurch gekennzeichnet, dass**
die funktionale Abhängigkeit des Biomarkers durch maschinelles Lernen aus Trainingsdaten bestimmt wurden, wobei der Biomarker eine gewichtete Summe von Amplituden-, Frequenz-, Phasen- und/oder Ortsinformationen des Messsignals darstellt.

5. System nach einem der vorherigen Ansprüche,
**gekennzeichnet durch** eine Feedback-Eingabeeinrichtung, wobei Feedback-Daten der Feedback-Eingabeeinrichtung bei der Berechnung des Biomarkers berücksichtigt werden.

6. System nach einem der vorherigen Ansprüche,
**dadurch gekennzeichnet, dass**
das Stimulationssignal einen Wechselstrom-Anteil und einen überlagerten Gleichstrom-Anteil aufweist.

## Claims

1. A system for non-invasive electric and/or magnetic neuronal stimulation, comprising:
- a signal generator for generating an alternating current stimulation signal,
- an application device configured for applying the stimulation signal in an area at or directly around the optic nerve,
- an outlet line for deriving a measurement signal,
- a biomarker calculation unit configured for calculating a biomarker based on the measurement signal, wherein the biomarker is a quantity of weighted parameters for which all of the data sets have characteristic properties or features for a diagnosis and/or a therapy target,
- a unit configured for implementating a stochastic optimization process for optimizing the value of the biomarker by variation of the stimulation signal.

2. The system according to claim 1,
**characterized by**
a noise generator for generating artificial noise as the stimulation signal.

3. The system according to any one of the preceding claims,
**characterized in that**
the stimulation signal is generated by a programmable function generator.

4. The system according to any one of the preceding claims,
**characterized in that**
the functional dependence of the biomarker was defined by machine learning from training data, wherein the biomarker constitutes a weighted sum of amplitude, frequency, phase and/or location information of the measurement signal.

5. The system according to any one of the preceding claims,
**characterized by** a feedback input device, wherein feedback data of the feedback input device is taken into account in calculating the biomarker.

6. The system according to any one of the preceding claims,
**characterized in that**
the stimulation signal has a proportion of alternating current and a superimposed proportion of direct current.

## Revendications

1. Système pour la stimulation neuronale non-invasive, électrique et/ou magnétique, comprenant :
- un générateur de signaux pour générer un signal de stimulation à courant alternatif,
- un dispositif d'application, configuré pour appliquer le signal de stimulation dans une zone sur ou directement autour du nerf optique,
- une dérivation pour dériver un signal de mesure,
- une unité de calcul de biomarqueur, configurée pour calculer un biomarqueur sur la base du signal de mesure,
le biomarqueur étant un ensemble de paramètres pondérés qui présentent des propriétés ou particularités caractéristiques pour tous les blocs de données par rapport à un diagnostic et à un objectif thérapeutique,
- une unité, configurée pour mettre en œuvre un procédé d'optimisation stochastique pour optimiser la valeur du biomarqueur par variation du signal de stimulation.

2. Système selon la revendication 1,
**caractérisé par**
un générateur de bruit pour générer un bruit artificiel comme signal de stimulation.

3. Système selon l'une des revendications précédentes,
**caractérisé en ce que**
le signal de stimulation est généré par un générateur de fonctions programmable.

4. Système selon l'une des revendications précédentes,
**caractérisé en ce que**
la dépendance fonctionnelle du biomarqueur est déterminée par apprentissage automatique à partir de données d'entraînement, le biomarqueur représentant une somme pondérée d'informations sur l'amplitude, la fréquence, la phase et/ou la localisation du signal de mesure.

5. Système selon l'une des revendications précédentes,
**caractérisé par** un dispositif d'entrée de rétroaction (feedback), dans lequel les données de rétroaction du dispositif d'entrée de rétroaction sont prises en compte dans le calcul du biomarqueur.

6. Système selon l'une des revendications précédentes,
**caractérisé en ce que**
le signal de stimulation comprend une composante de courant alternatif et une composante de courant continu superposée.
